# EUROPEAN PATENT SPECIFICATION

(11) **EP 2 651 481 B1**
(45) Date of publication and mention of the grant of the patent: **07.09.2016**
(21) Application number: 11811379.4
(22) Date of filing: 13.12.2011
(51) Int. Cl.: A61M 16/10, A61M 16/16

(54) **SYSTEM FOR ADMINISTERING HUMIDIFIED GAS TO A VENTILATED PATIENT**
SYSTEM ZUR VERABREICHUNG VON BEFEUCHTETEM GAS AN EINEN BEATMETEN PATIENTEN
SYSTÈME POUR ADMINISTRER UN GAZ HUMIDIFIÉ À UN PATIENT VENTILÉ

(30) Priority: 17.12.2010 US 201061424128 P
(43) Date of publication of application: 23.10.2013
(73) Proprietor: Koninklijke Philips N.V., 5656 AE Eindhoven (NL)
(72) Inventor: ARCILLA, Mabini, 5656 AE Eindhoven (NL); GARDE, Smita, 5656 AE Eindhoven (NL); AHMAD, Samir, 5656 AE Eindhoven (NL)
(74) Representative: Steffen, Thomas
(86) International application number: PCT/IB2011/055629
(87) International publication number: WO 2012/080941

(56) References cited:
- EP-A1- 1 066 850
- US-A- 6 095 505
- US-A- 6 102 037
- US-A1- 2002 170 559

## Description

### BACKGROUND

### 1. Field of the Disclosure

The invention relates to systems and methods for administering improved humidification of gas to a patient utilizing a ventilator.

### 2. Description of the Related Art

Conventional heated humidifiers are typically placed away from a patient to be treated (e.g., on a ventilator stand), which may present several problems (e.g., condensation in humidifier conduits). Solutions to these challenges may present additional challenges relating to humidity and temperature optimization for maximum effectiveness and maximum patient comfort. For example, conventional humidifiers may control the output of a heater plate but may lack mechanisms taking other aspects of the humidifier, or its applied use, into account. Furthermore, warning systems associated with conventional humidifiers to warn users of potential ineffectiveness, patient comfort, or other problems are deficient.

Other challenges posed by humidifier systems, regardless of their placement, may be addressed as well. For example, heated humidifiers used with ventilators are continually heating and humidifying dry gas from a ventilator, which is then delivered to a patient. The moisture supply, either through a water bag or with a water chamber will therefore be emptied over time. Other incidents may occur that inadequately deliver moisture to a humidifier. Furthermore, heat sources in heated humidifiers can become over-heated and/or otherwise malfunction. These scenarios may lead to delivery of inadequately heated and humidified gas to a patient.

Furthermore, the heat and humidification of ventilator gas delivered by conventional humidifiers is usually adjusted by controlling the temperature level of an associated heater. Hence, the relative humidity (RH) of gas delivered to a patient depends on the ambient temperature of the gas entering the humidifier and the heating that occurs in a heated wire circuit. This often leads to delivery of humidification that is less than optimal or less than 100% RH. The decreased level of humidity may lead to increased level of secretion retention in patients. Accordingly, caregivers must increase the temperature setting of the heater to allow for more water to be evaporated so as to increase in humidity levels. This increase in temperature may cause other problems (e.g., condensation in the patient tubing) and therefore may be an undesirable solution to inadequate humidity.

US 2002/0170559 A1 discloses a unit for adjusting humidification. This unit for adjusting humidification is characterized by an exothermic member provided on an outer surface thereof with multiple hollow fibers each having a peripheral wall with minute openings each of which is large enough to allow water vapor to permeate therethrough yet small enough to fail to permeate water therethrough.

EP 1066 850 A1 discloses a medical nebulizer with a reservoir for medical liquid, a capillary nozzle having a proximal end for receiving liquid from the reservoir and a distal end connectable to an inspiration gas flow path to deliver liquid droplets into an inspiration gas flow; pump means for supplying liquid from the reservoir through the capillary nozzle; and means for regulating the delivery of the liquid to a predetermined amount.

US 6,095,505 A discloses a patient-end humidifier including an evaporation module which has a contact chamber and a flash-resistant heat exchanger, a wicking layer on the heat exchanger, a liquid water flow controller, an electric resistance heater, and a breathing gas temperature controller.

These and other problems exist.

### SUMMARY

According to a first aspect, a humidifier system according to claim 1 is provided for humidifying gas that is delivered to a patient. The humidifier system comprises a water source, a humidifier unit, and a controller. The humidifier unit is disposed on a patient circuit that provides gas to a patient and may be located proximal to a patient interface of the patient circuit. The humidifier unit comprises a chamber that receives water from the water source, a heat source disposed within the chamber, a flow sensor that senses a flow of water provided to the chamber from the water source, and a temperature sensor that senses a temperature of humidified gas within the patient circuit. The controller has at least one processor that is configured to receive, from the flow sensor, flow data relating to water being provided to the chamber from the water source. The at least one processor is further configured to receive, from the temperature sensor, temperature data relating to the temperature of the humidified gas within the patient circuit. The at least one processor is further configured to adjust a flow of water being provided to the chamber by the water source based on the flow data and the temperature data.

In some embodiments, the humidifier unit may also include an ambient temperature sensor that senses an ambient temperature of an environment in which the humidifier system is placed, an input gas temperature sensor that senses a temperature of gas to be humidified by the humidifier unit, and/or an output humidity sensor that senses a humidity of the humidified gas within the patient circuit. In some embodiments, the controller may be a closed loop proportional-integral-derivative (PID) feedback controller that receives or determines one or more set points relating to a desired temperature of the humidified gas and/or a desired relative humidity of the humidified gas. The closed loop PID controller may receive process variable data from the temperature sensor, the ambient temperature sensor, the input gas temperature sensor, and/or the output humidity sensor. The closed loop PID controller may further adjust a heat output of the heat source and/or rate of water being provided to the chamber from the water source, using the process variable data to achieve the one or more received set points.

According to a second example a method is provided for humidifying gas delivered to a patient via a patient circuit having a humidifier unit disposed therein. The humidifier unit may be located proximal to a patient interface of the patient circuit. The method includes receiving flow data relating to water being provided from a water source to the humidifier unit and receiving temperature data relating to a temperature of humidified gas within the patient circuit. The method further includes adjusting a flow of water being provided to the humidifier unit by the water source based on the flow data and the temperature data.

In some examples , the method may be a closed loop feedback process that further includes receiving process variable data in the form of an ambient temperature of an environment in which the humidifier system is placed, a temperature of gas to be humidified by the humidifier unit, and/or a humidity of the humidified gas within the patient circuit. The method may also include receiving or determining one or more set points relating to one or more of a desired temperature of the humidified gas or a desired relative humidity of the humidified gas. The method may further comprise adjusting a heat output of the heat source or rate of water being provided to the humidifier unit from the water source using the process variable data to achieve the one or more received set points.

In some embodiments, a humidifier system may be provided for humidifying gas to be delivered to a patient. The system includes water source means for supplying a quantity of water, humidification means for humidifying gas to be provided to the patient, and controller means. The humidification means is disposed in a patient circuit that provides gas to a patient, proximal to a patient interface of the patient circuit. The humidification means includes chamber means for receiving water from the water source means, heat source means disposed within the chamber means for providing heat output, flow sensor means for sensing a flow of water provided to the chamber means from the water source means, and temperature sensor means for sensing a temperature of humidified gas within the patient circuit. The controller means recieves from the flow sensor means, flow data relating to water being provided to the chamber means from the water source means. The controller means further receives from the temperature sensor means, temperature data relating to the temperature of the humidified gas within the patient circuit. The controller means adjusts a flow of water being provided to the chamber means by the water source means and/or a heat output of the heat source means, based on the flow data and/or the temperature data.

In some embodiments, the humidification means may further comprise ambient temperature sensor means for sensing an ambient temperature of an environment in which the humidifier system is placed, input gas temperature sensor means for sensing a temperature of gas to be humidified by the humidification means, and/or output humidity sensor means that senses a humidity of the humidified gas within the patient circuit. The controller means may be a closed loop proportional-integral-derivative (PID) feedback controller means that receives or determines one or more set points relating to a desired temperature of the humidified gas and/or a desired relative humidity of the humidified gas. The closed loop PID controller means may also receive process variable data from the temperature sensor means, the ambient temperature sensor means, the input gas temperature sensor means, and/or the output humidity sensor means. The closed loop PID controller means may adjust a heat output of the heat source means and/or rate of water being provided to the chamber means from the water source means using the process variable data to achieve the one or more received set points.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. In one embodiment, the structural components illustrated herein are drawn to scale. It is to be expressly understood, however, that the drawings are for the purpose of illustration and description only and are not a limitation. In addition, it should be appreciated that structural features shown or described in any one embodiment herein can be used in other embodiments as well. It is to be expressly understood, however, that the drawings are for the purpose of illustration and description only and are not intended as a definition of limits. As used in the specification and in the claims, the singular form of "a", "an", and "the" include plural referents unless the context clearly dictates otherwise.

### BRIEF DESCRIPTION OF THE DRAWINGS

FIG. 1 illustrates an example of a humidifier system, according to various embodiments of the invention.
FIG. 2 illustrates a cross-section of an example of a humidifier unit, according to various embodiments of the invention.
FIG. 3 illustrates an example of a process for monitoring water flow within a humidifier system, according to various embodiments of the invention.
FIG. 4. illustrates an example of a process for monitoring humidified gas temperature within a humidifier system, according to various embodiments of the invention.
FIG. 5 illustrates an example of a humidifier system, according to various embodiments of the invention.
FIG. 6 illustrates a cross-section of an example of a humidifier unit, according to various embodiments of the invention.
FIG. 7 illustrates an example of a closed loop control module of a humidifier system, according to various embodiments of the invention.
FIG. 8 illustrates an example of process for utilizing a closed loop control module to control humidification of gas to be provided to a patient, according to various embodiments of the invention.

### DETAILED DESCRIPTION OF THE EXEMPLARY EMBODIMENTS

Embodiments described herein may deliver optimally heated and humidified gas to a patient via a patient circuit. In some implementations, such gas is delivered using a humidifier located proximally to a patient interface of a ventilation system. In some implementations, features addressing water leaks, obstructions, or other water/moisture flow problems may be used to prevent suboptimal humidifier operation and/or patient discomfort. In some implementations, features addressing issues relating to overheating or under heating of a heat source in the humidifier may also be used to prevent suboptimal operation and/or patient discomfort. In some implementations, alarms or notifications relating to issues relating to water flow, heating, or other issues may be enabled.

In some embodiments, a closed loop humidifier control system may be used with or without the other features disclosed herein. The closed loop control system may utilize temperature, humidity, and/or other sensor information as inputs to meet specified or calculated settings for gas temperature and/or humidity so as to further provide improved delivery of heated and humidified gas to a patient.

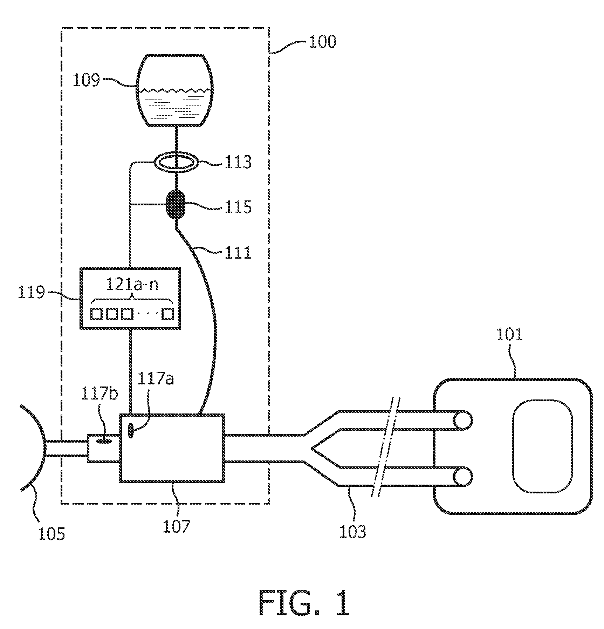

FIG. 1 illustrates a humidifier system 100, which is an example of a humidifier system that provides improved performance and patient comfort. Humidifier system 100 is illustrated as being used with ventilator 101. Ventilator 101 includes a patient circuit 103 that attached to or includes a patient interface 105 for delivering gas to a patient. Patient interface may include a nasal and/or oral mask, a nasal cannula, an invasive tube, and/or other interface with a patient's respiratory system. In some embodiments, the humidifier system includes a humidifier unit 107 that is disposed on or within patient circuit 103, proximal to patient interface. For example, in some implementations humidifier unit 107 is placed on patient circuit 103 between 6 to 8 inches from patient interface 105. Other distances may be used. Conventional humidifiers mounted on ventilator carts are typically placed 4 to 6 feet away from the patient.

Humidifier system 100 includes a water source 109 or other moisture source. In some embodiments, water source 109 may include container (e.g., bag, canister, etc) of fluid (e.g., water or other fluid suitable for humidifying gas to be delivered to a patient). In some implementations, water source 109 is connected to humidification unit 107 via a supply tube 111, which may be or include flexible tubing or other conduit capable of transporting fluid from water source 109 to humidifier unit 107. In some implementations, a fluid connection between water source 109 and supply tube 111 may be such that water (or other fluid) enters supply tube drop by drop (e.g., through a drip chamber).

In some embodiments, humidifier system 100 includes a flow sensor 113 that detects the movement of fluid from water source 109 to humidifier unit 107. In one example, flow sensor 113 may be an optical sensor that is disposed along supply tube 111. In some embodiments, the optical sensor may include an light emitting diode (LED) and a photodetector arranged on a clear or otherwise translucent portion of supply tube 111 so that fluid passing through supply tube 111 passes between the LED and the photodetector, enabling the photodetector to detect fluid flow. In embodiments wherein fluid exits water source 109 in drops, the optical sensor may detect the movement of said drops through supply tube 111 and therefore determine fluid flow through supply tube 111. In some embodiments, flow sensor 113 may be or include flow sensors other than optical flow sensors.

In some embodiments, humidifier system 100 may include a valve 115 that controls fluid flow between water source 109 and humidifier unit 107. For example, valve 115 may include a "pinch" valve located on supply tube 111 that may be used to pinch close supply tube 111, thereby preventing or otherwise controlling fluid flow therethrough.

In some embodiments, humidifier system 100 includes one or more temperature sensors. For example, FIG. 1 illustrates a plurality of temperature sensors 117 disposed on humidifier unit 107. Temperature sensors 117 may be, for example, thermocouple sensors or other type of temperature sensors.

In some embodiments, other sensors may be provided in similar or other locations of the humidification system (including those sensors described elsewhere herein). For example, additional temperature or humidity sensors may be located along patient circuit 103. Other sensors such as, for example, flow sensors or pressure sensors, may also be utilized.

FIG. 1 illustrates a proximally placed humidifier system 100, wherein humidification of gas to be delivered to a patient occurs at humidifier unit 107. In some implementations, humidifier unit 107 may utilize a membrane-based humidifier. A membrane-based humidifier may include a heat source that heats a small volume of water delivered by a water source (e.g., water source 109) to a water chamber separated from main gas flow path by a hydrophobic membrane (that will not allow liquid water to pass therethrough). As the water evaporates, water vapors pass through the membrane into the gas path, humidifying the incoming dry gas. In some implementations, the water may be fed into the water chamber through gravity feed, pump, or by other methods. In some implementations, the drip or flow of water into the water chamber may depend on the rate of evaporation of water in the water chamber.

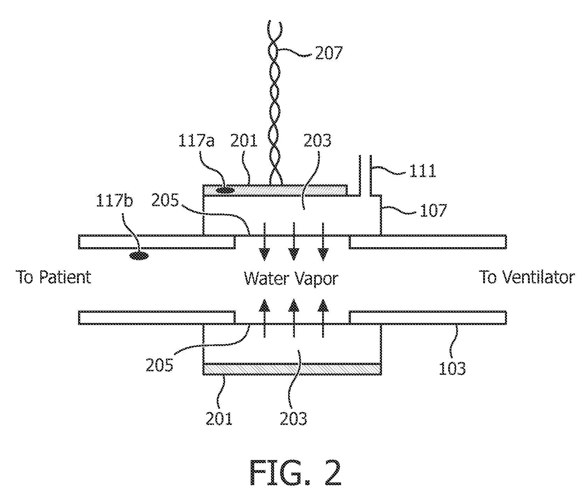

FIG. 2 illustrates a cross-section of humidifier unit 107 located on patient circuit 103. Humidifier unit 107 may encircle a portion of patient circuit 103 and may include a heat source 201 which also encircles patient circuit 103. In some embodiments, heat source 201 may comprise one or more discrete segments that provide heat rather than encircling patient circuit 103. Heat source 201 may be for example, a PTC (positive temperature coefficient) ceramic heater element, insulated etched foil heater, insulated wound wire heater or other heating element suitable for use in medical applications. Heat source 201 may include connectors 207, which may be or include wires or other connectors providing power and/or an operative connection to controller 119. Humidifier unit 107 includes a water chamber 203 (which, as illustrated in FIG. 2, also surrounds a portion of patient circuit 103) to which moisture from water source 109 is provided (e.g., via supply tube 111). Humidifier unit 107 may also include membrane 205, which encircles a portion of patient circuit 103 and separates water chamber 203 from fluid communication with patient circuit 103. Membrane 205 may comprise a hydrophobic membrane that is impermeable to liquid water but permeable to water vapor (e.g., a membrane made using a polyterafluoroethylene [PTFE] base material). As such, liquid water cannot enter patient circuit 103 from water chamber 203, but vaporized water can enter, thereby humidifying the gas in patient circuit 103.

FIG. 2 also illustrates temperature sensors 117a and 117b. Temperature sensor 117a may be positioned so as to measure the temperature of heat source 201. Temperature sensor 117b is be positioned so as to mention the temperature of heated gas in the patient circuit. In some embodiments, one or both of temperature sensors 117a or 117b may be thermocouple sensors. However, in some embodiments, other sensors may be used.

Referring back to FIG. 1, humidifier system 100 includes a controller 119. In some embodiments, controller 119 may include a computer-implemented device having one or more micro-processors, associated memory, and/or other computer components for performing various computing tasks, including receipt of data, processing data, decision making, issuance of commands/instructions/signals, and/or other related tasks. Controller 119 may be operatively connected with the various sensors and valves that form part of the humidifier system (e.g., optical sensor 113, pinch valve 115, temperature sensors 117a and 117b, and/or any other elements). Controller 119 may include one or more modules 121a-121n that configure/instruct the one or more processors of controller 119 to perform one or more features or functions relating to humidification of a gas, including receiving or determining a desired fluid flow/delivery rate, a desired temperature for humidified gas, a desired humidity of humidified gas, and/or other levels/settings. Modules 121a-121n may also configure/enable the one or more processors of controller 119 to receive data regarding flow or passage of fluid to humidifier unit 107 from water source 109, control actuation of valve 115 (and therefore control fluid flow or passage from water source 109 to humidifier unit 107), receive data regarding the temperature of humidified gas from one or more temperature sensors, receive data regarding the temperature of a heat source, receive data regarding the humidity of humidified gas, control the heat output of a heat source, perform one or more determinations/calculations as described herein, and/or other features.

For example, one of modules 121a-121n may include a moisture control module. The moisture control module may receive (e.g., from sensor 113) data regarding the flow of water from water source 109 to water chamber 203 (or extrapolate data regarding such flow from information received from sensor 113). In some embodiments, the humidifier system may enable a user (e.g., a medical professional or other caregiver/operator) to set a flow rate. As such, in some implementations controller 119 may include an interface (e.g., keypad, touch screen, etc.) by which to receive such desired flow rate from a user. In some embodiments, the moisture control module or other part of controller 121 may determine a desired or target flow rate (e.g., using a desired humidity level and/or other inputs).

The moisture control module may adjust the flow of water to water chamber 203 by actuating valve 115. As such, if flow rate is to be slowed or stopped, the moisture control module may cause valve 115 to close (completely or partially) and stop or reduce flow in supply tube 111. Conversely, to commence or increase flow of water to moisture chamber 203, the moisture control module may cause valve 115 to open (completely or partially) and permit or increase the flow of water through supply tube 111.

The moisture control module may not only enable control of flow of water to water chamber 203, but may also enable detection of one or more problems or alarm conditions relating to water flow/levels in the humidifier system. For example, if water source 109 becomes empty and flow of water from water source 109 ceases, the moisture control module may detect this condition (using e.g., sensor 113) and trigger an alarm. The alarm may be an external alarm that notifies a user (e.g., medical caregiver, etc.). The external alarm may include a visual and/or auditory signal to the user so as to notify the user that water source 109 is empty. In some embodiments, the alarm may be internal to controller 121 (instead of or in addition to notifying users of the condition) so that controller may take an automatic action in response thereto (e.g., shutting down/adjusting heat source 201, switching to a different water source, etc). This and other alarms may be useful, as the patient is no longer receiving gas having the desired humidity. Furthermore, without such alarms, heat source 201 may continue to produce heat which, in the absence of water, may damage the humidifier system or may elevate the heat of gas in the patient circuit beyond recommended or desired levels.

Similar to the detection of an empty water source 109, the moisture control module may detect other conditions relating to moisture delivery to humidifier system 100. For example, the humidifier system may develop a leak in its water delivery system (e.g., a leak in membrane 205, water chamber 203, supply tube, etc.). The moisture control module may detect this leak. For example, the drip or flow of water into water chamber 203 may depend on the rate of evaporation of the water in water chamber 203. As such, when a rupture or leak in membrane 205 occurs, fluid flow will increase. Sensor 113 may provide the moisture control module with data indicating that fluid flow exceeds a determined level of fluid flow necessary for desired humidification) and may cause an external alarm to notify a user and/or an internal alarm to shut down heat source 201 and/or other portion of humidification system 100 (or even ventilator 101).

The moisture control module may also enable detection of blockages in supply tube 111, or humidifier unit 107 (e.g., sensor 113 may detect a back up of fluid or slowed fluid flow in supply tube 111 while valve 115 is open) and may similarly cause an external alarm to notify a user or an internal alarm to take other action (e.g., shut down heat source 201).

In some embodiments, one of modules 121a-121n may include a heat control module. The heat control module may receive data regarding the temperature of heat source 201 (e.g., from sensor 117a), the temperature of gas in the patient circuit and (e.g., from sensor 117b), and/or other data. In some embodiments, the humidifier system may enable a user to set a heat output. As such, in some embodiments, controller 119 may include an interface (e.g., keypad, touch screen, etc.) by which to receive such desired heat output settings from a user. In some embodiments, the heat control module or other part of controller 119 may determine a desired or target heat output for heat source 201, (e.g., using a desired humidity level and/or other factors). Using this determined or received desired heat output, heat control module may modulate the energy sent to heat source 201 so as to achieve the desired heat output.

As described herein the heat control module may work in concert with other portions of controller to instantiate external or internal alarms (e.g., if the temperature of gas in patient circuit 103 is higher or lower than desired) and/or carry out actions in response to alarms or other commands relating to humidification of gas in patient circuit 103. For example, if problems relating to water source 109 or other parts of the moisture supply for humidifier system 100 make it desirable to lower heat output, raise heat out put, or shut down heat source 201, the heat control module may enable such action.

Modules 121a-121n may also include modules for providing additional features, including the closed loop control features described herein. Furthermore, as discussed herein, the modules and alarms provided for by one or more module 121a-121n (or modules 521 a-521 n), may be integrated with ventilator alarms such that commands or alarms from ventilator 101 may cause actions by one or more module 121a-121n (or modules 521a-521n). Conversely, commands or alarms from one or more modules 121 a-121 n (or modules 521a-521n) may cause actions by ventilator 101 or other equipment.

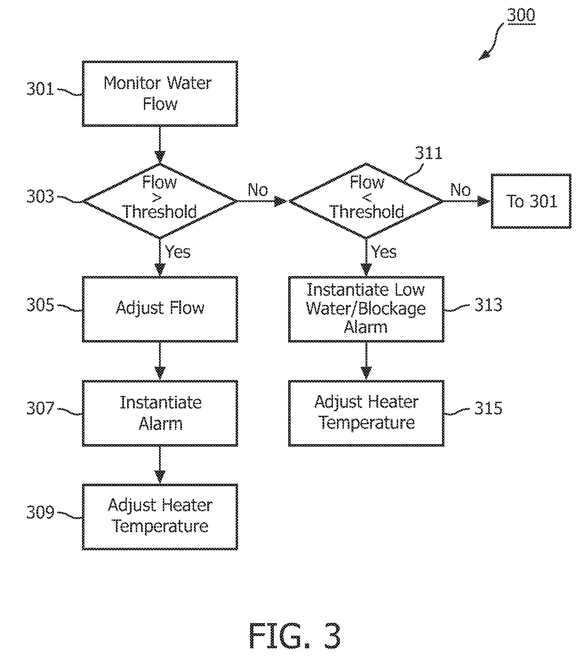

FIG. 3. illustrates a process 300, which is an example of a process for optimizing water flow within a humidifier system. Process 300 may be performed by a moisture control module or other portion of controller 119. Process 300 includes an operation 301 wherein water flow to a humidifier is monitored. In some implementations, this includes a flow sensor (e.g., sensor 113) monitoring flow through a supply tube (e.g., supply tube 111). In an operation 303, it is determined whether the measured flow exceeds a first predetermined flow threshold. In some implementations, the first predetermined flow threshold may be selected as an indicator of whether a leak exists in the humidifier system.

If it is determined that the flow exceeds the first predetermined threshold, the flow of water in the humidifier system may be adjusted in an operation 305. This may include utilizing a valve (e.g., valve 115) to stop flow of water to the humidifier unit. In some embodiments, flow may be reduced, rather than stopped.

Process 300 may proceed to an operation 307, wherein an alarm relating to a water leak may be instantiated. As discussed herein, the alarm may be an internal or external alarm. Process 300 may then proceed to an operation 309, wherein a temperature of a heater (e.g., heat source 201) is adjusted. For example, of a leak is detected and water flow is stopped, heat source 201 may be turned off so as to avoid overheating the heat source or delivering gas to a patient that is higher than a desired temperature. In some instances, the temperature of a heater may be reduced rather then shut down altogether.

If, in operation 303, it is determined that the water flow does not exceed the first predetermined threshold, process 300 may proceed to an operation 311, wherein it is determined whether the flow is less then a second predetermined threshold. Flow less than the second predetermined flow threshold may indicate that the water supply (e.g., water source 109) is empty or that there is a blockage in the water supply system (e.g., supply line 111). If it is determined that the flow is less than the second predetermined threshold, process 300 may proceed to an operation 313, wherein a low water or blockage alarm is instantiated. As discussed herein, the alarm may be an internal or external alarm. Process 300 may then proceed to an operation 315, wherein the temperature of the heater (e.g., heat source 201) is adjusted. For example, if the water supply is empty, heat source 201 may be shut off so as to avoid so as to avoid overheating the heat source or delivering gas to a patient that is higher than a desired temperature. In some instances, the temperature of heater may be reduced rather than shut off altogether.

If, in operation 311, it is determined that flow is not less than the second predetermined threshold, operation may return to operation 301 wherein water flow rate is monitored.

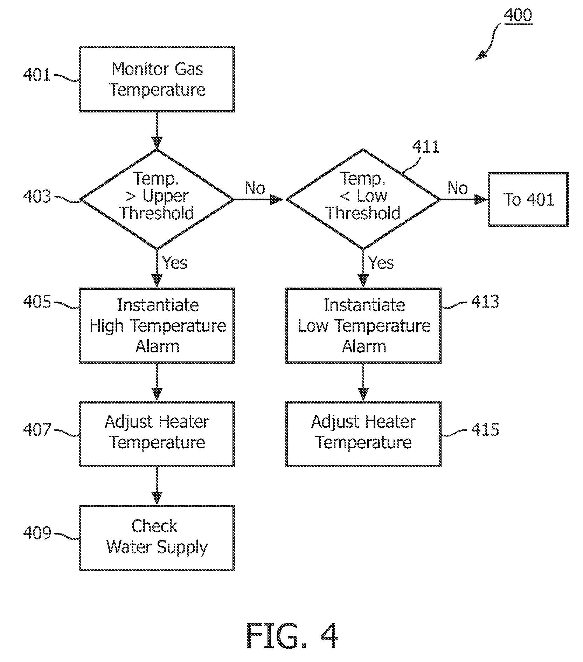

FIG. 4 illustrates a process 400, which is an example of a process for optimizing heat source temperature within a humidifier system. In some implementations, process 400 may be performed by a heat control module or other of controller 119. Process 400 includes an operation wherein the temperature of humidified gas to be delivered to a patient is monitored. For example, temperature sensor 117b of humidifier system 100 may monitor gas humidified by humidification unit 107. In an operation 403, it is determined whether the monitored temperature exceeds a predetermined high temperature threshold. A temperature higher then the high temperature threshold may deliver gas of a suboptimal temperature and/or humidity to a patient. If the temperature exceeds the predetermined high temperature threshold, process 400 may proceed to an operation 405, wherein a high temperature alarm is instantiated. As discussed herein, this alarm may be an internal alarm or an external alarm. Process 400 may then proceed to an operation 407 wherein a heat source may be adjusted. For example, the output of heat source 201 may be reduced so as to achieve a desired temperature (or humidity) of humidified gas to be provided to a patient. In some instances the output of heat source 201 may be ceased altogether.

Process 400 may then proceed to an operation 409 wherein the water supply of the humidifier system may be checked. For example, increased temperature may be a result of a problem in the water supply (e.g., an empty water source) and therefore examined. Operation 409 may include a water control module performing process 300 or other process to determine whether a problem with the water supply exists.

If, in operation 403, it is determined that the temperature does not exceed the predetermined high temperature threshold, process 400 may proceed to an operation 411, wherein it is determined whether the temperature is less than a predetermined low temperature threshold. A temperature lower then the low temperature threshold may deliver gas of a suboptimal temperature and/or humidity to the patient. If it is determined that the temperature is lower than the predetermined low temperature threshold process 400 may proceed to operation 413, wherein a low temperature alarm may be instantiated. As discussed herein this alarm may be an internal or external alarm. Process 400 may then proceed to an operation 415, wherein the heater temperature may be adjusted. For example, the output of heat source 201 may be increased.

If, in operation 411, it is determined that the temperature is not less than the predetermined low temperature threshold, process 400 may return to operation 401, therein the temperature of humidified gas is monitored further.

In some humidifier applications, the temperature of the dry gas entering a conventional humidifier may be relatively high (e.g., due to high ambient temperature or when an associated ventilator heats the gas). As such, humidifier heaters may not have to heat the incoming gas long enough to reach a set temperature, as less heating is needed. Less heating at the point of humidification may causes less evaporative moisture to be added to the dry gas and thus a relative humidity that is less than desired. Also, some typical humidifier heaters use heated wire circuits. These heated wires can increase the gas temperature to be higher than the humidifier output to overcome some problems with conventional humidifiers such as, for example, condensation. These and/or other issues relating to conventional systems may obscure knowledge of the precise amount of absolute or relative humidity delivered to the patient. Inadequate humidification is typically only found when increased retention of secretions is observed.

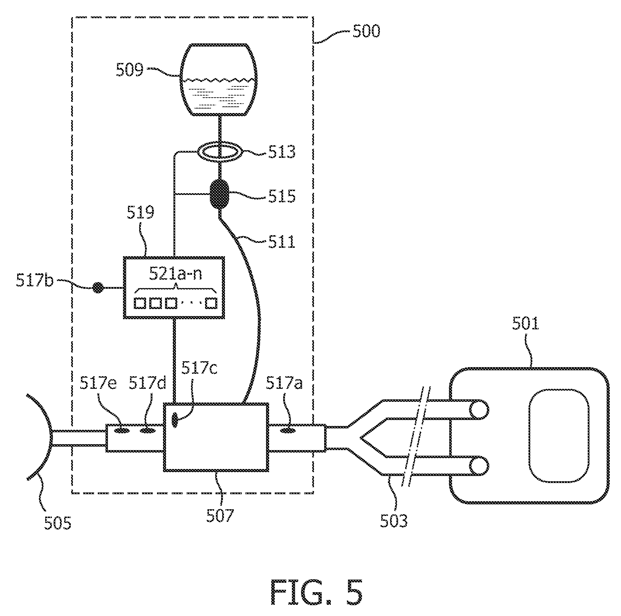

In some implementations, a humidifier system may include closed loop control of heating and humidification of gas for delivery to a patient, which may provide more accurate and controllable monitoring and manipulation of temperature and humidity. FIG. 5 illustrates a humidifier system 500, which may be used in conjunction with a ventilator 501 and its associated patient circuit 503 (having a patient interface 505). Humidifier system 500 includes humidifier unit 507, water source 509, supply tube 511, flow sensor 513, valve 515 (e.g., pinch valve), one or more sensors 517 (including one or more temperature and humidity sensors, as detailed herein), and controller 519.

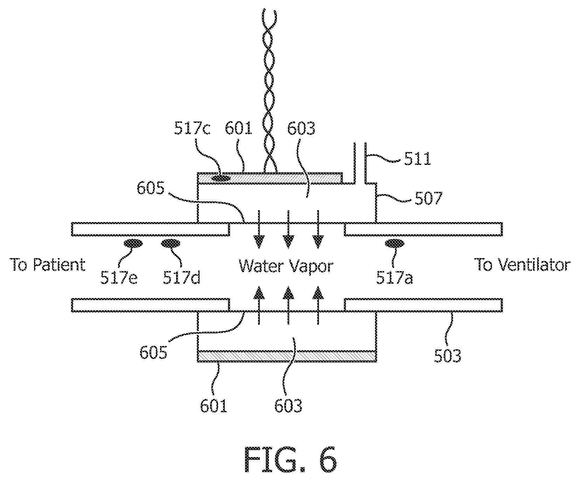

FIG. 6 illustrates membrane-based humidifier unit 507, including heat source 601, water chamber 603, and membrane 605. Heat source 601 may include connectors 607, which may be or include wires or other connectors providing power and an operative connection to controller 519. FIGS. 5 and 6 also illustrates the numerous sensors that may be used with closed lop control of temperature and humidity of gas provided to a patient using patient circuit 503. For example, humidifier unit 507 includes temperature sensor 517a, which may be a thermocouple or other temperature sensor that is located so as to sample the temperature of the gas being fed into humidifier unit 507 (i.e., upstream of humidifier unit 507 during patient inhalation). This enables determination of the temperature of gas prior to humidification so that the amount of moisture and/or heat necessary to provide gas of a desired humidity and temperature to the patient may be accurately determined.

Humidifier system 500 may include an ambient temperature sensor 517b, which may be a thermocouple or other temperature sensor positioned to sample the temperature of the environment in which humidification system 500 is placed. Humidifier unit 507 may also include a temperature sensor 517c which may include a thermocouple or other temperature sensor that is located so as to determine the temperature of heat source 601. Humidifier unit 507 includes a temperature sensor 517d, which may be a thermocouple or other temperature sensor that is located so as to sample the temperature of humidified gas in patient circuit 503 (i.e., down stream of humidifier unit 507 during patient inhalation). Sampling of gas after humidification, especially in humidifiers located proximally to a patient interface, provides the benefit of more accurate determination of the temperature of gas supplied to the respiratory system of a patient. This enables more optimal adjustment of gas temperature and humidity mad may avoid temperatures that cause patient discomfort.

Humidifier unit 507 may also include a humidity sensor 517e which may be located so as to sample the humidity of humidified gas to be provided to the patient (i.e., downstream of humidification unit 507 during patient inhalation). In some embodiments, humidity sensor 517e may comprise a capacitive sensor whose capacitance changes with the relative humidity of the gas (it may also include a temperature sensing ability so as to provide accurate humidity readings).

Controller 519 may include a computer-implemented device having one or more micro-processors, associated memory, and/or other computer components to perform various computing tasks, including receipt of data, processing data, decision making, issuance of commands/instructions/signals, and/or other related tasks. Controller 519 may be operatively connected with the various sensors and valves that form part of the humidifier system (e.g., flow sensor 513, valve 515, temperature sensors 517a-d, humidity sensor 517e, and any other sensors present). Controller 519 may include one or more modules 321 a-321 n that enable the one or more processors to perform one or more features or functions relating to optimized humidification and heating of a gas provided to a patient (including those the same as or similar to moisture control module and heat control module described herein in association with humidifier system 100).

Modules 521 a-521 n may include a closed loop control module 521 a. Closed loop control module 521 a may configure/instruct the one or more processors of controller 519 to deliver optimal levels of heating and humidification of gas for delivery to a patient. For example, closed loop control module 521 a may utilize feedback control (similar to a proportional-integral-derivative [PID] controller) to adjust the heater output of heat source 601 and the flow of water to water chamber 603 (e.g., using valve 515) so as to control both temperature and humidification levels of humidified gas. Similar to a PID controller, closed loop control module 521 a may utilize one or more process variables (i.e., inputs) and one or more set points (i.e., settings) to achieve the aforementioned control.

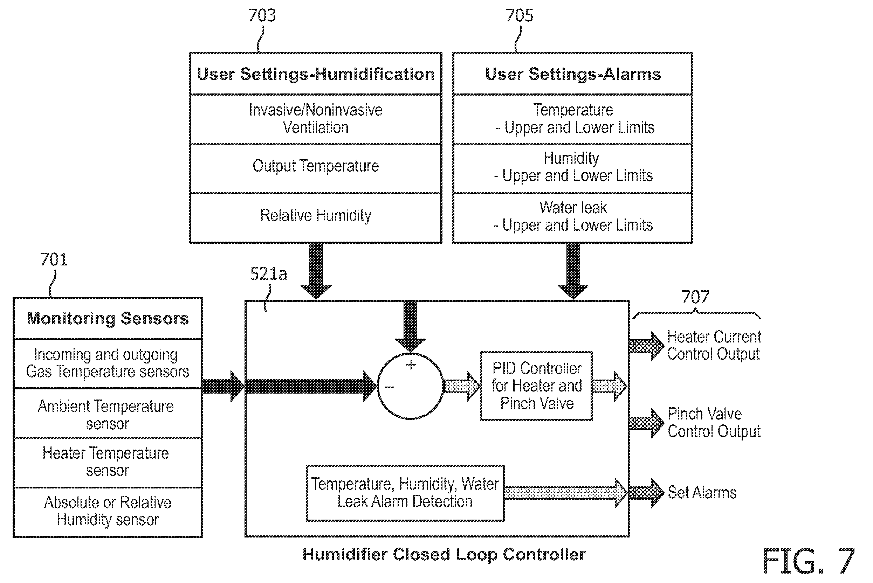

Fig. 7 illustrates closed loop control module 521 a. Closed loop control module 521 may include, as process variables/inputs one or more of, sensor input 701, which may include data regarding temperature of the gas to be humidified (e.g., from temperature sensor 517a), the ambient temperature of the environment in which humidification system 500 is placed (e.g., from ambient temperature sensor 517b), data regarding the temperature of humidified gas (e.g., from temperature sensor 517d), data regarding the humidity of humidified gas (e.g., an absolute and/or relative humidity from humidity sensor 517e), data regarding the output of heat source 601 (e.g., from heat source sensor 517c), data regarding the flow of water through supply tube 511 (e.g., from flow sensor 513), and/or other inputs.

Closed loop control module 521 a may also receive or determine one or more set points/settings 703. One or more of settings 703 may be received from a user (e.g., via an interface, as described herein) computed, and/or otherwise determined. Settings 703 may include a set temperature of humidified gas to be provided to the patient and a relative humidity of the humidified gas to be provided to the patient. Closed loop control module may utilize the sensor inputs (e.g., ambient temperature, input gas temperature, etc.) and adjust the heat output of heat source 601 and the flow of water (using valve 515) so as to achieve the desired output heat and relative humidity as dictated by settings 703. In some implementations, settings 703 may include whether ventilation to the patient is invasive (e.g., via intubation) or noninvasive (via mask, nasal cannula, etc) as the level of humidification required for these treatment methods may be different (e.g., 44 mg/L at 37 degrees C for invasive and 25-30 mg/L at 31 degrees C for noninvasive ventilation). Closed loop control module 521 a may also receive other settings such as alarm settings 705. Alarm settings may include settings that trigger one or more internal or external alarms based on upper and/or lower temperature limits for humidified gas, upper and lower humidity limits, upper and lower water flow limits, and/or other alarm settings (see e.g., FIGS 1-4).

As described herein, closed loop control module 501 may utilize these inputs and settings to formulate one or more outputs 707. Outputs 707 may include a heater current output for controlling heat output from heat source 601 as well as a valve control output for controlling flow of water to humidification unit 507 (e.g., using valve 515).

Closed loop control modules may be used with proximal humidifiers and traditional humidifiers equipped with the appropriate sensor inputs and control algorithms.

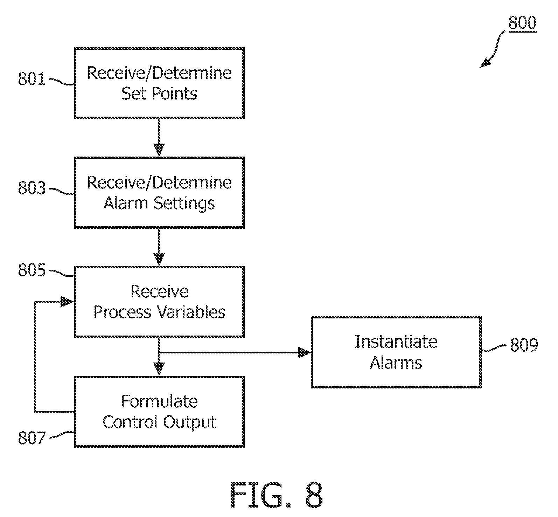

FIG. 8 illustrates a method 800, which is an example of a method for utilizing a closed loop control module to control humidification of gas to be provided to a patient. Method 800 includes an operation 801, wherein one or more set points/settings (e.g., set points 703) are determined or received (e.g., from a user) at the closed loop control module. As described herein these set points/settings may include an output temperature for gas to be provided to a patient, an output relative humidity to be provided to a patient, whether associated ventilation is invasive or noninvasive, or other set points.

In an operation 803, the closed loop control module may receive or determine one or more alarm settings (e.g., alarm settings 705). The alarm settings may include predetermined high and/or low temperature, humidity, or water leak parameters that dictate instantiation of internal or external alarms as discussed herein.

In an operation 805, the closed loop control module receives one or more process variables from various sensors in a humidification/ventilation system. These process variables may include data regarding the temperature of incoming and outgoing gas, an ambient temperature of the environment in which the humidifier is placed, a temperature of a heat source of the humidifier, the absolute or relative humidity of humidified gas, and/or other process variables.

In an operation 807, the closed loop control module may utilize the process variables to formulate output signals (i.e., control outputs) for the heat source and water flow within the humidifier so as to achieve the temperature and humidity settings. The precise algorithm used may depend on the type and value of process inputs received and output settings sought. However it will be appreciated that, for example, certain relationships between input process variables and the resulting output signals may exist.

For example, when a desired temperature and relative humidity of humidified gas is determined or provided, the humidifier system (e.g., system 500) monitors the temperature (e.g., via temperature sensor 517a) of the incoming gas (e.g., from ventilator 501). Based on the difference in temperature between the incoming gas and the outgoing gas (e.g., as measured by temperature sensor 517d), the closed loop control module will cause an output to a heater (e.g., heat source 601) that will proportionally increase (or decrease if error is negative) the level and rate of change of the heater current. The increase (or decrease) in heater current will consequently change the temperature and the humidity of the outgoing gas (which is measured by e.g., temperature sensor 517d and 517e). If a humidity of outgoing gas (e.g., as measured by humidity sensor 517e) is less than the desired humidity, the closed loop control module will proportionately increase the flow of water to the humidifier (e.g., utilize valve 515 to increase the flow of water through supply tube 511 to humidifier unit 507) and also increase the heater current in order to be able to generate higher levels of humidity in the gas flow through the humidifier. Based on the ambient temperature of the environment in which the humidifier system is placed (e.g., as measured by ambient temperature sensor 517b), the closed loop control module may adjust the heater current required to heat the supplied water. As an example, a higher ambient temperature will result in a higher temperature of incoming water to the humidifier (e.g., humidifier unit 507) and the closed loop control module will increase the heater current to a smaller amount than when the ambient temperature is lower. When the temperature and the humidity of the outgoing gas (e.g., as measured by 517d and 517e) are close to the desired settings, the closed loop control module will change the heater current and the water flow to the heater in smaller steps at a slower rate to continue maintaining the desired humidity and/or temperature in the outgoing gas. Monitoring the temperature of the heater (e.g., heat source 601, via sensor 517c) may also be used to ensure the output generated by the closed loop control module translates into the appropriate/desired heat output.

Process 800 may then return to operation 805, wherein the one or more process variables may be received again. As such, the closed loop control module continuously monitor the process variables and provide updated outputs so as to achieve the set points. In an operation 809, when one or more of the process variables/inputs exceed or drops below a certain specified threshold (e.g., alarm settings 705), one or more alarms may be instantiated.

Some of the implementations described herein may be used for adult, pediatric and neonatal ventilation therapies that require the humidification and heating of gases for home and hospital use. The therapies may include invasive ventilation, noninvasive ventilation, high flow oxygen therapy, neonatal CPAP, home CPAP for adult OSA, and/or other therapies. The embodiments, components, and methods described herein may be used in other patient care and or humidification applications.

The system and methods described herein are provided as examples only. Those having skill in the art will appreciate that the systems described herein may work with various system configurations. Accordingly, more or less of the aforementioned system components may be used and/or combined in various implementations. It should also be understood that various software modules that are utilized to accomplish the functionalities described herein may be maintained on other components than those described herein. In some implementations, as would be appreciated, the functionalities described herein may be implemented in various combinations of hardware and/or firmware, in addition to, or instead of, software. The processes described herein may utilize more or less of the described operations and the order of operations may be altered as would be appreciated.

Embodiments further include non-transitory computer readable media (e.g., discs, memory sticks, hard disks, or other volatile or non-volatile storage media) having computer executable instructions thereon that cause/configure/instruct one or more processors to perform some or all of the features and functions described herein.

Details included herein are for the purpose of illustration based on what is currently considered to be the most practical and preferred embodiments, it is to be understood that such detail is solely for that purpose and that the scope of this specification is not limited to the disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A humidifier system for humidifying gas delivered to a patient, comprising:
a water source (109, 509);
a humidifier unit (107, 507) disposed on a patient circuit (103, 503) that provides gas to a patient, the humidifier unit (107, 507) located proximal to a patient interface (105, 505) of the patient circuit (103, 503), the humidifier unit (107, 507) comprising:
a chamber (203, 603) that receives water from the water source (109, 509),
a heat source (201, 601) disposed within the chamber (203, 603),
a flow sensor (113, 513) that senses a flow of water provided to the chamber (203, 603) from the water source (109, 509), and
a temperature sensor (117b, 517d) that senses a temperature of humidified gas within the patient circuit (103, 503); and
a controller (119, 519) having at least one processor configured to:
receive, from the flow sensor (113, 513), flow data relating to water being provided to the chamber (203, 603) from the water source (109, 509),
receive, from the temperature sensor (117b, 517d), temperature data relating to the temperature of the humidified gas within the patient circuit (103, 503), and
**characterized in that** the at least one processor is further configured to:
adjust a flow of water being provided to the chamber (203, 603) by the water source (109, 509) based on the flow data and temperature data.

2. The humidifier system of claim 1, wherein the humidifier unit (107, 507) further comprises a hydrophobic membrane (205, 605) that separates the chamber (203, 603) from the patient circuit (103, 503), and wherein the hydrophobic membrane (205, 605) prevents liquid water from entering the patient circuit (103, 503) from the chamber (203, 603) but permits water vapor to enter the patient circuit (103, 503) from the chamber (203, 603).

3. The humidifier system of claim 1, wherein the controller (119, 519) having at least one processor is further configured to:
adjust a heat output of the heat source (201, 601) based on one or more of the flow data or temperature data, and
wherein the humidifier unit (507) further comprises one or more of:
an ambient temperature sensor (517b) that senses an ambient temperature of an environment in which the humidifier system is placed,
an input gas temperature sensor (517a) that senses a temperature of gas to be humidified by the humidifier unit (507), or
an output humidity sensor (517e) that senses a humidity of the humidified gas within the patient circuit (503),
and wherein adjustment of one or more of the flow of water being provided to the chamber (603) by the water source (509) or the heat output of the heat source (601) is further based on one or more of:
the ambient temperature,
the temperature of the gas to be humidified, or
the humidity of the humidified gas.

4. The humidifier system of claim 1, wherein the humidifier unit (507) further comprises one or more of:
an ambient temperature sensor (517b) that senses an ambient temperature of an environment in which the humidifier system is placed,
an input gas temperature sensor (517a) that senses a temperature of gas to be humidified by the humidifier unit (507), or
an output humidity sensor (517e) that senses a humidity of the humidified gas within the patient circuit
and wherein the controller (519) is a proportional-integral-derivative (PID) feedback controller that:
receives or determines one or more set points (703) relating to one or more of a desired temperature of the humidified gas or a desired relative humidity of the humidified gas,
receives process variable data (701) from one or more of:
the temperature sensor (517d),
the ambient temperature sensor (517b),
the input gas temperature sensor (517a), or
the output humidity sensor (517e), and
adjusts one or more of a heat output of the heat source (601) or rate of water being provided to the chamber (603) from the water source (509) using the process variable data (701) to achieve the one or more received set points (703).

5. The humidifier system of claim 1, wherein the at least one processor is further configured to generate one or more alarms when one or more of:
a rate of water being provided to the chamber (203, 603) from the water source (109, 509) exceeds a first predetermined threshold,
a rate of water being provided to the chamber (203, 603) from the water source (109, 509) is less than a second predetermined threshold,
the temperature of the humidified gas provided to the patient exceeds a predetermined high temperature threshold, or
the temperature of the humidified gas provided to the patient is less than a predetermined low temperature threshold.

## Patentansprüche

1. Befeuchtersystem zum Befeuchten von einem Patienten zuzuführendem Gas, wobei das Befeuchtersystem Folgendes umfasst:
eine Wasserquelle (109, 509);
eine Befeuchtereinheit (107, 507), die an einem Patientenkreislauf (103, 503) angeordnet ist, der einem Patienten Gas bereitstellt, wobei die Befeuchtereinheit (107, 507) proximal zu einer Patientenschnittstelle (105, 505) des Patientenkreislaufs (103, 503) angeordnet ist, wobei die Befeuchtereinheit (107, 507) Folgendes umfasst:
eine Kammer (203, 603), die Wasser von der Wasserquelle (109, 509) empfängt,
eine Wärmequelle (201, 601), die innerhalb der Kammer (203, 603) angeordnet ist,
einen Durchflusssensor (113, 513), der eine der Kammer (203, 603) von der Wasserquelle (109, 509) aus bereitgestellte Wasserströmung erfasst, und
einen Temperatursensor (117b, 517d), der eine Temperatur des befeuchteten Gases innerhalb des Patientenkreislaufs (103, 503) erfasst; und
eine Steuereinheit (119, 519) mit mindestens einem Prozessor, die konfiguriert ist zum:
Empfangen von Strömungsdaten von dem Durchflusssensor (113, 513), die sich auf das der Kammer (203, 603) von der Wasserquelle (109, 509) aus bereitgestellte Wasser beziehen,
Empfangen von Temperaturdaten von dem Temperatursensor (117b, 517d), die sich auf die Temperatur des befeuchteten Gases innerhalb des Patientenkreislaufs (103, 503) beziehen, und
**dadurch gekennzeichnet ist, dass** mindestens ein Prozessor weiterhin konfiguriert ist zum:
Anpassen der Wasserströmung, die der Kammer (203, 603) durch die Wasserquelle (109, 509) bereitgestellt wird, basierend auf den Strömungsdaten und den Temperaturdaten.

2. Befeuchtersystem nach Anspruch 1, wobei die Befeuchtereinheit (107, 507) weiterhin eine hydrophobe Membran (205, 605) umfasst, die die Kammer (203, 603) von dem Patientenkreislauf (103, 503) trennt, und wobei die hydrophobe Membran (205, 605) verhindert, dass flüssiges Wasser von der Kammer (203, 603) in den Patientenkreislauf (103, 503) eintritt, aber erlaubt, dass Wasserdampf von der Kammer (203, 603) in den Patientenkreislauf (103, 503) eintritt.

3. Befeuchtersystem nach Anspruch 1, wobei die Steuereinheit (119,519) mit mindestens einem Prozessor konfiguriert ist zum:
Anpassen der Wärmeabgabe der Wärmequelle (201, 601) basierend auf einem oder mehreren von den Strömungsdaten oder den Temperaturdaten, und
wobei die Befeuchtereinheit (507) weiterhin eines oder mehrere der folgenden Elemente umfasst:
einen Umgebungstemperatursensor (517b), der eine Umgebungstemperatur einer Umgebung erfasst, in der das Befeuchtersystem aufgestellt ist,
einen Eingangsgastemperatursensor (517a), der eine Temperatur des durch die Befeuchtereinheit (507) zu befeuchtenden Gases erfasst, oder
einen Ausgangsfeuchtigkeitssensor (517e), der eine Feuchtigkeit des befeuchteten Gasis innerhalb des Patientenkreislaufs (503) erfasst,
und wobei die Anpassung von einem oder mehreren, der der Kammer (603) durch die Wasserquelle (509) zugeführten Wasserströmung oder der Wärmeabgabe der Wärmequelle (601), weiterhin basiert auf einem oder mehreren von:
der Umgebungstemperatur,
der Temperatur des zu befeuchtenden Gases, oder
der Feuchtigkeit des befeuchteten Gases.

4. Befeuchtersystem nach Anspruch 1, wobei die Befeuchtereinheit (507) weiterhin eines oder mehrere von Folgendem umfasst:
einen Umgebungstemperatursensor (517b), der eine Umgebungstemperatur einer Umgebung erfasst, in der das Befeuchtersystem aufgestellt ist,
einen Eingangsgastemperatursensor (517a), der eine Temperatur des durch die Befeuchtereinheit (507) zu befeuchtenden Gases erfasst, oder
einen Ausgangsfeuchtigkeitssensor (517e), der eine Feuchtigkeit des befeuchteten Gases innerhalb des Patientenkreislaufs erfasst,
und wobei die Steuereinheit (519) ein Proportional-Integral-Differential-(PID) Feedback-Regler ist, der
einen oder mehrere Sollwerte (703) in Bezug auf eines oder mehrere von einer gewünschten Temperatur des befeuchteten Gases oder einer gewünschten relativen Feuchtigkeit des befeuchteten Gases empfängt oder bestimmt,
Prozessvariablendaten (701) empfängt von einem oder mehreren von:
dem Temperatursensor (517d),
dem Umgebungstemperatursensor (517b),
dem Eingangsgastemperatursensor (517a), oder
dem Ausgangsfeuchtigkeitssensor (517e), und
eine oder mehrere von einer Wärmeausgabe der Wärmequelle (601) oder Rate des der Kammer (603) von der Wasserquelle (509) bereitgestellten Wassers unter Verwendung der Prozessvariablendaten (701) anpasst, um den einen oder mehrere empfangene Sollwerte (703) zu erreichen.

5. Befeuchtersystem nach Anspruch 1, wobei der mindestens eine Prozessor weiterhin konfiguriert ist, um einen oder mehrere Alarme zu erzeugen bei einem oder mehreren von:
eine Rate des der Kammer (203, 603) von der Wasserquelle (109, 509) bereitgestellten Wassers überschreitet einen ersten vorgegebenen Schwellenwert,
eine Rate des der Kammer (203, 603) von der Wasserquelle (109, 509) bereitgestellten Wassers unterschreitet einen zweiten vorgegebenen Schwellenwert,
die Temperatur des dem Patienten zugeführten befeuchteten Gases überschreitet einen vorgegebenen hohen Temperaturschwellenwert, oder
die Temperatur des dem Patienten zugeführten befeuchteten Gases unterschreitet einen vorgegebenen niedrigen Temperaturschwellenwert.

## Revendications

1. Système d'humidification pour humidifier un gaz délivré à un patient, comprenant :
une source d'eau (109, 509) ;
une unité d'humidification (107, 507) disposée sur un circuit de patient (103, 503) qui fournit du gaz à un patient, l'unité d'humidification (107, 507) étant située près d'une interface de patient (105, 505) du circuit de patient (103, 503), l'unité d'humidification (107, 507) comprenant :
une chambre (203, 603) qui reçoit de l'eau de la source d'eau (109, 509),
une source de chaleur (201, 601) disposée à l'intérieur de la chambre (203, 603),
un capteur d'écoulement (113, 513) qui détecte un écoulement d'eau fourni à la chambre (203, 603) à partir de la source d'eau (109, 509), et
un capteur de température (117b, 517d) qui détecte une température de gaz humidifié à l'intérieur du circuit de patient (103, 503) ; et
un régulateur (119, 519) ayant au moins un processeur configuré pour :
recevoir, du capteur d'écoulement (113, 513), des données d'écoulement concernant l'eau étant fournie à la chambre (203, 603) à partir de la source d'eau (109, 509),
recevoir, du capteur de température (117b, 517d), des données de température concernant la température du gaz humidifié à l'intérieur du circuit de patient (103, 503), et
**caractérisé en ce que** l'au moins un processeur est configuré en outre pour :
régler un écoulement d'eau étant fourni à la chambre (203, 603) par la source d'eau (109, 509) sur la base des données d'écoulement et des données de température.

2. Système d'humidification selon la revendication 1, dans lequel l'unité d'humidification (107, 507) comprend en outre une membrane hydrophobe (205, 605) qui sépare la chambre (203, 603) du circuit de patient (103, 503), et dans lequel la membrane hydrophobe (205, 605) empêche l'eau liquide de pénétrer dans le circuit de patient (103, 503) à partir de la chambre (203, 603) mais permet à la vapeur d'eau de pénétrer dans le circuit de patient (103, 503) à partir de la chambre (203, 603).

3. Système d'humidification selon la revendication 1, dans lequel le régulateur (119, 519) ayant au moins un processeur est en outre configuré pour :
régler une sortie de chaleur de la source de chaleur (201, 601) sur la base d'une ou de plusieurs des données d'écoulement ou des données de température, et
dans lequel l'unité d'humidification (507) comprend en outre un ou plusieurs parmi :
un capteur de température ambiante (517b) qui détecte une température ambiante d'un environnement dans lequel est placé le système d'humidification,
un capteur de température de gaz d'entrée (517a) qui détecte une température de gaz devant être humidifié par l'unité d'humidification (507), ou
un capteur d'humidité de sortie (517e) qui détecte une humidité du gaz humidifié à l'intérieur du circuit de patient (503),
et dans lequel le réglage d'un ou plusieurs de l'écoulement d'eau étant fourni à la chambre (603) par la source d'eau (509) ou de la sortie de chaleur de la source de chaleur (601) est en outre basé sur un ou plusieurs d'entre :
la température ambiante,
la température du gaz devant être humidifié, ou
l'humidité du gaz humidifié.

4. Système d'humidification selon la revendication 1, dans lequel l'unité d'humidification (507) comprend en outre un ou plusieurs parmi :
un capteur de température ambiante (517b) qui détecte une température ambiante d'un environnement dans lequel est placé le système d'humidification,
un capteur de température de gaz d'entrée (517a) qui détecte une température de gaz devant être humidifié par l'unité d'humidification (507), ou
un capteur d'humidité de sortie (517e) qui détecte une humidité du gaz humidifié à l'intérieur du circuit de patient
et dans lequel le régulateur (519) est un régulateur à réaction proportionnel, intégral, dérivé (PID) qui :
reçoit ou détermine un ou plusieurs points de consigne (703) concernant un ou plusieurs d'une température souhaitée du gaz humidifié ou d'une humidité relative souhaitée du gaz humidifié,
reçoit des données variables de processus (701) d'un ou plusieurs parmi :
le capteur de température (517d),
le capteur de température ambiante (517b),
le capteur de température de gaz d'entrée (517a), ou
le capteur d'humidité de sortie (517e), et
règle un ou plusieurs d'une sortie de chaleur de la source de chaleur (601) ou d'un débit d'eau étant fourni à la chambre (603) à partir de la source d'eau (509) à l'aide des données variables de processus (701) pour atteindre le ou les points de consigne reçus (703).

5. Système d'humidification selon la revendication 1, dans lequel l'au moins un processeur est configuré en outre pour générer une ou plusieurs alarmes lorsqu'un ou plusieurs parmi :
un débit d'eau étant fourni à la chambre (203, 603) à partir de la source d'eau (109, 509) dépasse un premier seuil prédéterminé,
un débit d'eau étant fourni à la chambre (203, 603) à partir de la source d'eau (109, 509) est inférieur à un second seuil prédéterminé,
la température du gaz humidifié fourni au patient dépasse un seuil de température élevé prédéterminé, ou
la température du gaz humidifié fourni au patient est inférieure à un seuil de température bas prédéterminé.
